(19) Europäisches Patentamt
European Patent Office
Office européen des brevets

(11) Numéro de publication: **0 037 327**
**B1**

(12) # FASCICULE DE BREVET EUROPEEN

(45) Date de publication du fascicule du brevet: **28.09.83**

(51) Int. Cl.$^3$: **C 07 C 147/107**

(21) Numéro de dépôt: **81400474.3**

(22) Date de dépôt: **25.03.81**

(54) **Nouveaux dérivés substitués de l'acide 3-formyl 4-méthyl pentanoique, leur préparation et leur application à la préparation d'acide 3-formyl but-3-èn-1-oique substitué.**

(30) Priorité: **28.03.80 FR 8006978**

(43) Date de publication de la demande:
**07.10.81 Bulletin 81/40**

(45) Mention de la délivrance du brevet:
**28.09.83 Bulletin 83/39**

(84) Etats contractants désignés:
**AT BE CH DE GB IT LI LU NL SE**

(56) Documents cités:
**US - A - 3 445 499**

(73) Titulaire: **ROUSSEL-UCLAF**
**35, boulevard des Invalides**
**F-75007 Paris (FR)**

(72) Inventeur: **Martel, Jacques**
**15, rue Douvillez**
**F-93140 Bondy (FR)**
Inventeur: **Tessier, Jean**
**30, rue Jean Moulin**
**F-94300 Vincennes (FR)**
Inventeur: **Demoute, Jean-Pierre**
**249bis, rue de Rosny**
**F-93100 Montreuil sous Bois (FR)**

(74) Mandataire: **Tonnellier, Marie-José et al,**
**102, route de Noisy Boîte postale no 9**
**F-93230 Romainville (FR)**

**EP 0 037 327 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England

Nouveaux dérivés substitués de l'acide 3-formyl 4-méthyl pentanoïque, leur préparation et leur application à la préparation d'acide 3-formyl but-3-èn-1-oïque substitué

La présente invention concerne de nouveaux dérivés substitués de l'acide 3-formyl 4-méthyl pentanoïque, leur préparation et leur application à la préparation d'acide 3-formyl but-3-èn-1-oïque substitué.

L'invention a pour objet les dérivés de l'acide 3-formyl 4-méthyl pentanoïque de formule (I):

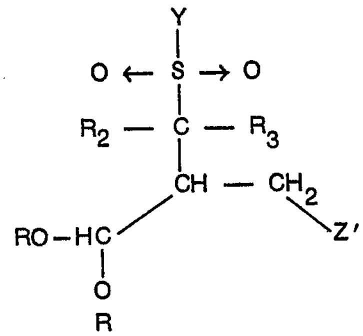

(I)

dans laquelle Y représente un reste aromatique, $R_2$ et $R_3$ représentent un radical alcoyle linéaire ou ramifié comportant de 1 à 4 atomes de carbone ou $R_2$ et $R_3$ représentent ensemble avec l'atome de carbone auquel ils sont liés un homocycle carboné comportant de 3 à 6 atomes de carbone, R représente un radical alcoyle comportant de 1 à 6 atomes de carbone ou bien les 2 radicaux R représentent ensemble une chaîne polyméthylène comportant 2 ou 3 atomes de carbone, et Z' représente un groupement $CO_2H$ ou un groupement Z, Z représentant un radical cyano ou un groupement $CO_2$ $R_1$, $R_1$ représentant un radical alcoyle comportant de 1 à 6 atomes de carbone.

Dans la formule I,

— Y représente par exemple un radical phényle, xylyle ou, de préférence, tolyle;

— $R_2$ et $R_3$ représentent un radical méthyle, éthyle, propyle, isopropyle, butyle, sec-butyle ou tert-butyle, ou $R_2$ et $R_3$ forment ensemble avec l'atome de carbone auxquels ils sont liés, un cycle cyclopropyle, cyclobutyle, cyclopentyle ou cyclohexyle;

— R représente par exemple un radical méthyle, éthyle, propyle ou butyle ou les radicaux R forment avec les atomes d'oxygène auxquels ils sont liés un radical éthylène dioxy ou triméthylène dioxy;

— $R_1$ représente par exemple un radical méthyle, éthyle, propyle, isopropyle, butyle, sec-butyle ou tert-butyle.

L'invention a ainsi pour objet les dérivés de formule I répondant à la formule ($I_A$):

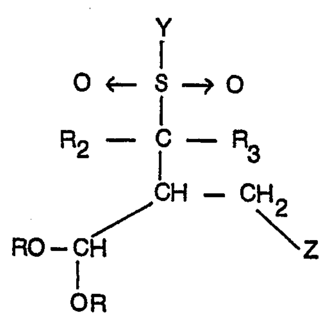

($I_A$)

dans laquelle Y, Z, R, $R_2$ et $R_3$ conservent les mêmes significations que précédemment, ainsi que les dérivés de formule I répondant à la formule ($I_B$):

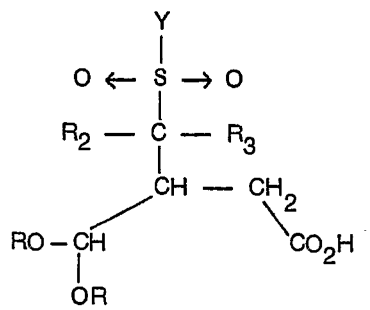

$(I_B)$

dans laquelle Y, R, $R_2$ et $R_3$ conservent les mêmes significations que précédemment.

L'invention a également pour objet un procédé de préparation des dérivés de formule I, telle que définie précédemment, caractérisé en ce que l'on fait réagir, en présence d'une base forte, au sein d'un solvant polaire, un composé de formule générale (II):

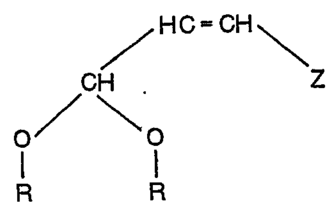

(II)

dans laquelle Z et R conservent les mêmes significations que précédemment, avec une sulfone de formule (III):

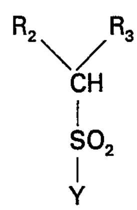

(III)

dans laquelle $R_2$, $R_3$ et Y conservent les mêmes significations que précédemment, pour obtenir un composé de formule générale $(I_A)$:

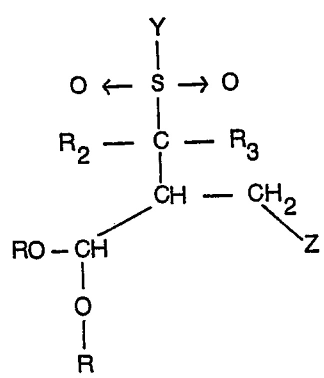

$(I_A)$

dans laquelle Y, Z, R, $R_2$ et $R_3$ conservent les mêmes significations que précédemment, puis, si désiré, fait réagir sur le composé de formule $(I_A)$

—*soit*, lorsque Z représente un groupe —$CO_2R_1$, dans lequel $R_1$ est tel que défini précédemment, un agent basique susceptible de saponifier le groupement —$CO_2R_1$ pour obtenir l'acide correspondant de formule $(I_B)$:

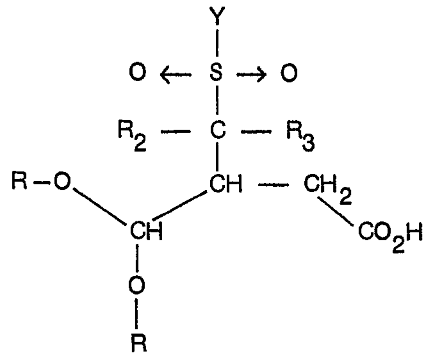

($I_B$)

dans laquelle Y, R, $R_2$ et $R_3$ conservent les significations précitées,

— *soit,* lorsque Z représente un groupement cyano, un agent d'hydrolyse susceptible de transformer le cyano en acide, pour obtenir également l'acide correspondant de formule ($I_B$).

Dans le procédé selon l'invention, de préférence, la base forte en présence de laquelle on fait réagir le composé (II) avec le composé (III) est choisie dans le groupe constitué par les alcoolates alcalins, les hydrures alcalins, les amidures alcalins, le phényl lithium et les alcoyls lithiens; le solvant polaire au sein duquel on fait réagir le composé de formule (II) et le composé de formule (III) est choisi dans le groupe constitué par le diméthylsulfoxyde, le diméthoxyéthane, le diméthylformamide, le tétrahydrofuranne, l'hexaméthylphosphorotriamide utilisé pur ou en mélange avec des hydrocarbures aromatiques monocycliques ou des cycloalcanes et un mélange de ces solvants; l'agent basique susceptible de saponifier le groupement $-CO_2R_1$ pour obtenir le composé de formule ($I_B$) est un hydroxyde alcalin en solution hydroalcoolique; l'agent d'hydrolyse du groupement cyano du composé de formule ($I_A$) est également un hydroxyde alcalin en solution hydroalcoolique mais que l'on utilise en solution plus concentrée et dans un temps plus long que pour la saponification du groupement ester.

L'invention a également pour objet l'application des composés de formule I, telle que définie précédemment, à la préparation d'acide 3-formyl but-3-èn-1-oïque substitué, application qui consiste à transformer le composé de formule ($I_A$) en composé de formule ($I_B$), selon le procédé défini précédemment, et qui est caractérisé en ce que l'on fait réagir sur le composé de formule ($I_B$) un agent acide susceptible d'hydrolyser la fonction cétal de la molécule, obtient ainsi un composé de formule (IV):

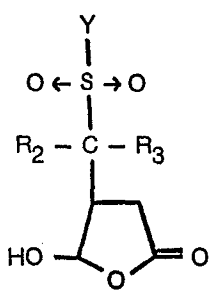

(IV)

et la soumet à l'action d'un agent basique pour obtenir l'acide 3-formyl 4-$R_2R_3$ but-3-èn-1-oïque.

Dans l'application selon l'invention, de préférence, l'agent acide susceptible d'hydrolyser la fonction cétal du composé ($I_B$) est un acide fort utilisé à l'état de traces, choisi dans le groupe constitué par l'acide sulfurique, l'acide chlorhydrique et l'acide paratoluènesulfonique, ledit acide fort étant utilisé en milieu acétonique et aqueux et l'agent basique que l'on fait agir sur le composé de formule (IV) est un carbonate alcalin.

L'acide 3-formyl 4-$R_2$ $R_3$ but-3-èn-1-oïque obtenu selon l'invention est utile pour préparer des composés de formule:

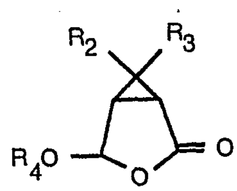

(V)

formule dans laquelle $R_4$ représente un atome d'hydrogène ou le reste d'un alcool $R_1OH$, par exemple selon le procédé décrit pour $R_2$=$R_3$=$CH_3$ dans le demande de brevet européen publiée sous le n° 0 023 849 le 11 février 1981.

Un exemple de préparation d'un composé de formule (V) est décrit ci-après dans la partie expérimentale.

les composés (V) servent notamment à préparer les acides d'esters insecticides très actifs.

Les exemples ci-après illustrent l'invention sans la limiter.

Exemple 1

Acide 3-formyl 4-méthyl pent-3-ène-1-oïque.

*Stade A:* 4-paratolyl sulfonyl 4-méthyl 3-diméthoxy méthyl pentane nitrile.

Dans 40 cm3 de tétrahydrofuranne, on introduit 7,79 g de paratolyl isopropyl sulfone, refroidit à —20, —30°C, ajoute 22,5 cm3 de solution benzenique de n-butyl lithium 1,75M, agite, refroidit à —60, —70°C et introduit une solution de 5 g de diméthyl acétal $\beta$-cyanoacroléine dans 30 cm3 de tétrahydrofuranne, agite pendant 40 minutes à —60, —70°C puis pendant 1 heure 30 minutes à —30/—35°C, verse le mélange réactionnel sur une solution aqueuse glacée de dihydrogénophosphate de sodium, extrait au benzène concentre à sec sous pression réduite, chromatographie le produit brut sur silice en éluant au mélange benzène-acétate d'éthyle (9—1) et obtient 8,33 g de 4-paratolyl sulfonyl 4-méthyl 3-diméthoxyméthyl pentane nitrile, F = 99°C.

*Spectre IR* (chloroforme)
absorption à 2240 $cm^{-1}$ attribuée au C≡N
absorption à 1593 $cm^{-1}$—1490 $cm^{-1}$ attribuées aux noyaux aromatiques,
absorption à 1307 $cm^{-1}$—1295 $cm^{-1}$—1142 $cm^{-1}$ attribuées aux $SO_2$,

*Spectre de RMN* (deutérochloroforme)
pic à 1,35 p p m attribué aux méthyles géminés,
pic à 2,46 p p m attribué aux hydrogènes du méthyl du tolyle
pics à 2,75 p p m attribués aux hydrogènes en $\alpha$ et $\beta$ du nitrile,
pic à 3,4 p p m attribué aux hydrogènes des méthoxy,
pic à 4,7 p p m attribué à l'hydrogène en $\alpha$ des méthoxy,
pics à 7,3, 7,5 p p m attribués aux hydrogènes aromatiques en position 3 et 5 du radical paratolyl,
pics à 7,7, 7,9 p p m attribués aux hydrogènes en position 2 et 6 du paratolyle.

*Stade B:* Acide 4-paratolyl sulfonyl 4-méthyl 3-diméthoxy méthyl pentanoïque.

Dans 200 cm3 d'une solution à 50% en volume de soude 2N dans l'alcool éthylique, on introduit 4,98 g de 4-paratolyl sulfonyl 4-méthyl 3-diméthoxy méthyl pentane nitrile, porte le mélange réactionnel au reflux, l'y maintient pendant 110 heures, extrait au chlorure de méthylène les produits non acides, acidifie à pH 2 la phase aqueuse avec de l'acide oxalique, extrait la phase aqueuse ainsi acidifiée au chlorure de méthylène, concentre à sec la phase organique sous pression réduite et obtient 4,5 g d'acide 4-paratolyl sulfonyl 4-méthyl 3-diméthoxy méthyl pentanoïque. F = 144°C.

*Spectre IR* (Chloroforme)
Absorption à 3510 $cm^{-1}$ attribuée à l'OH acide (monomère + dimère)
Absorption à 1745 $cm^{-1}$ attribuée à

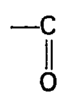

de l'acide monomère,
Absorption à 1711 $cm^{-1}$ attribuée à

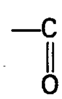

de l'acide dimère,
Absorption à 1606 $cm^{-1}$, 1425 $cm^{-1}$ attribuée aux noyaux aromatiques,
Absorption à 1289, 1148, 1128 $cm^{-1}$ attribuée à —$SO_2$.

*Spectre RMN* (deuterochloroforme)
pics à 1,31—1,36 p p m attribués aux méthyles géminés,
pic à 2,46 p p m attribué aux hydrogènes du méthyle du paratolyle,
pics à 2,66—3,08 p p m attribués aux hydrogènes en $\alpha$ et $\beta$ du carboxyle,
pics à 3,29—3,38 p p m attribués aux hydrogènes des méthoxy,
pics à 4,4—4,46 p p m attribués à l'hydrogène en $\alpha$ des méthoxy,

pics à 7,3—7,4 p p m attribués aux hydrogènes en 3 et en 5 du paratolyl,
pics à 7,75—7,9 p p m attribués aux hydrogènes en 2 et 6 du paratolyle.

*Stade C:* dl trans 4(2-paratolyl sulfonyl prop-2-yl) 5-hydroxy tétrahydrofuran-2-one.

Dans un mélange de 14 cm3 d'acétone et de 20 cm3 de solution aqueuse d'acide chlorhydrique N, on introduit 1 g d'acide 4-paratolyl sulfonyl 4-méthyl 3-diméthoxy méthyl pentanoïque, porte le mélange réactionnel au reflux, maintient le reflux pendant 4 heures, extrait au chlorure de méthylène, concentre la phase organique à sec par distillation sous pression réduite et obtient 0,793 g de dl trans 4-(2-paratolyl sulfonyl prop-2-yl) 5-hydroxy tétrahydrofuran-2-one.

*Spectre IR* (chloroforme)
Absorption à 3580 $cm^{-1}$ attribuée à l'OH associé,
Absorption à 1785 $cm^{-1}$ attribuée à

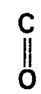

de la $\gamma$ lactone
Absorptions à 1595 $cm^{-1}$—1488 $cm^{-1}$ attribuées aux noyaux aromatiques,
Absorption à 1310 $cm^{-1}$, 1300 $cm^{-1}$, 1125 $cm^{-1}$ attribuées au $SO_2$.

*Stade D:* Acide 3-formyl 4-méthyl pent-3-ène 1-oïque.

Dans une solution de 20 cm3 d'eau et de 2,4 cm3 de méthanol, on introduit 0,788 g de dl trans 4(2-paratolyl sulfonyl prop-2-yl) 5-hydroxy tétrahydrofuran-2-one, agite a —5°C, ajoute 0,790 g de carbonate de sodium, agite pendant 2 heures à 20°C, extrait à l'éther éthylique les impuretés organiques non acides, acidifie la phase aqueuse à pH = 3,5 à l'aide d'une solution chlorhydrique aqueuse 1N, extrait la phase aqueuse au chloroforme, concentre la phase organique à sec par distillation sous pression réduite et obtient 0,310 g d'acide 3-formyl-4-méthyl pent-3-èn-1-oïque. F = 102°C.

*Spectre de RMN* (deutérochloroforme)
pics à 2,0 et 2,26 p p m attribués aux méthyles,
pic à 3,38 p p m attribué aux hydrogènes en 2,
pic à 10,13 p p m attribué à l'hydrogène du formyl.

Exemple II

Acide 3-formyl 4-méthyl pent-3-ène-1-oïque.

*Stade A:* 4-paratolyl sulfonyl 4-méthyl 3-diméthoxy méthyl pentanoate de méthyle.

Dans 13 cm3 de tétrahydrofuran, on introduit 1,24 g de paratolyl isopropyl sulfone, introduit à —70°C 3,4 cm3 d'une solution de butyl lithium dans l'hexane à 1,95 mole/litre, agite pendant 15 minutes, ajoute lentement 1 g de l'ester méthylique de l'acide (E) 4,4-diméthoxy 2-buténoïque en solution dans 20 cm3 de tétrahydrofuran, agite pendant 1 heure à —70°C, verse le mélange réactionnel sur une solution aqueuse de phosphate monosodique, extrait au chlorure de méthylène, sèche, concentre à sec sous pression réduite, chromatographie le résidu sur silice en éluant avec un mélange d'éther de pétrole (eb 40—70°C) et d'éther éthylique, cristallise dans l'éther isopropylique et obtient 1,1 g de 4-paratolyl sulfonyl 4-méthyl 3-diméthoxy méthyl pentanoate de méthyle F = 110°C.

*Spectre IR* (chloroforme)
absorption à 1732 $cm^{-1}$ attribuée à C=O,
absorption à 1600 $cm^{-1}$, 1495 $cm^{-1}$ attribuée au noyau aromatique,
absorption à 1310 $cm^{-1}$, 1301 $cm^{-1}$, 1250 $cm^{-1}$ attribuée à $SO_2$,
absorption à 690 $cm^{-1}$ attribuée à

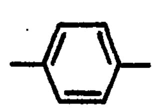

*Spectre de RMN* (deutérochloroforme)
pics à 1,31—1,35 p p m attribués aux hydrogènes des $CH_3$ géminés,
pic à 2,45 p p m attribué aux hydrogènes du méthyle du paratolyl,
pics à 3,26—3,35 p p m attribués aux hydrogènes des méthoxy,
pic à 3,6 p p m attribué aux hydrogènes de l'ester méthylique,

pics à 4,3—4,4 p p m attribués à hydrogène en $\alpha$ des méthoxy,
pics à 7,3—7,4 p p m et 7,45—7,3 p p m attribués aux hydrogènes du noyau aromatique,

*Stade B:* Acide 4-paratolyl sulfonyl 4-méthyl 3-diméthoxy méthyl pentanoïque.

Dans un mélange de 20 cm3 de solution aqueuse 2N de soude et de 20 cm3 d'éthanol on introduit 1 g de 4-paratolyl sulfonyl 4-méthyl 3-diméthoxy méthyl pentanoate de méthyle, porte le mélange réactionnel au reflux, l'y maintient pendant 17 heures, refroidit, verse dans l'eau, extrait au chlorure de méthylène, concentre à sec et obtient l'acide 4-paratolyl sulfonyl 4-méthyl 3-diméthoxy méthyl pentanoïque.

*Stades C et D:*

Ils sont identiques à ceux de l'exemple 1 et conduisent à l'acide 3-formyl 4-méthyl pent-3-ène 1-oïque de mêmes constantes que celui de l'exemple 1.

L'exemple suivant illustre le procédé de préparation d'un composé de formule (V) au départ de l'acide 3-formyl 4-méthyl pent-3-èn-1-oïque.

Exemple III

6,6-diméthyl 4-hydroxy 3-oxa bicyclo [3.1.0] hexan 2-one (dl)

*Stade A:* 4-(2-chloro-prop 2-yl) 5-hydroxy tétrahydrofuran 2-one dl trans.

Sous un courant d'acide chlorhydrique anhydre, on agite 1 g d'acide 3-formyl 4-méthyl pent-3-ène 1-oïque obtenu à l'exemple précédent, 25 cm3 d'éther éthylique anhydre et 1 g de chlorure de lithium sec pendant 2 heures à —30°C, puis 2 heures à 0°C, arrête le courant d'acide chlorhydrique et continue l'agitation 48 heures à température ambiante. Après 54 heures de contact, on verse le mélange réactionnel dans de l'eau glacée, décante, extrait au benzène, sèche, concentre à sec sous pression réduite, obtient 1,1 g de produit brut, le chromatographie sur silice en éluant par un mélange benzène-acétate d'éthyle (1—1), et obtient 545 mg d'un produit qui cristallise F = 80°C.

*Spectre RMN*

pics à 1,55 et 1,66 p p m attribués aux hydrogènes des méthyles,
pics à 2,42 à 2,92 p p m attribués aux hydrogènes en position 3 et 4 du cycle,
pics à 5,82—5,89 p p m attribués à l'hydrogène en 5 du cycle,
pic à 3,83 p p m attribué à l'hydrogène de l'hydroxyle.

*Stade B:* 4-(2-chloro-prop-2-yl) 5-[(3-phénoxyphényl) méthoxy] tétrahydrofuran 2-one dl trans.

On agite pendant 19 heures à température ambiante 393 mg du produit obtenu précédemment, 668 g d'alcool métaphénoxybenzylique, 20 mg d'acide paratoluènesulfonique et 5 cm3 de benzène, neutralise avec un peu de bicarbonate de sodium, sèche, concentre à sec sous pression réduite, obtient 1,18 g de produit brut, le chromatographie sur silice en éluant par du benzène, récupère 467 mg de produit, qui, recristallisé dans l'éther de pétrole fond à environ 50°C.

*Spectre RMN*

pics à 1,5 et 1,55 p p m attribués aux hydrogènes des méthyles,
pics à 2,55—2,72 p p m attribués aux hydrogènes en position 3 et 4 du cyclopentyle,
pics à 5,52—5,56 p p m attribués à l'hydrogène en position 5 du cyclopentyle,
pics à 6,92—7,5 p p m attribués aux hydrogènes des noyaux aromatiques,
pics à 4,47—4,66 et 4,77—4,97 p p m attribués aux hydrogènes du méthylène benzylique.

*Stade C:* dl 6,6-diméthyl 4-hydroxy 3-oxa bicyclo [3.1.0] hexan 2-one.

On refroidit, vers —20°C, 0,55 cm3 d'une solution 1M de diisopropylamine dans du tétrahydrofuranne et 5 cm3 de tétrahydrofuranne, ajoute 0,25 cm3 d'une solution de n-butyllithium 2M dans du cyclohexane, laisse remonter la température à 0°C, puis refroidit à —60°C—70°C, ajoute en une seule fois 180 mg du produit obtenu au stade B. Après deux heures sous agitation en laissant la température remonter jusqu'à 0°C, on maintient une heure à cette température, verse le mélange réactionnel dans de l'acide chlorhydrique 2N glacé et laisse 17 heures à 20°C sous vive agitation, décante, extrait au chloroforme, sèche, concentre à sec, reprend le résidu obtenu par un mélange éther isopropylique-éther de pétrole, extrait à l'eau, concentre à sec sous pression réduite la phase aqueuse pour obtenir 30 mg de produit cristallisé. F = 80°C.

**Revendications pour les Etats contractants: BE CH DE GB IT LI LU NL SE**

1. Les composés de formule:

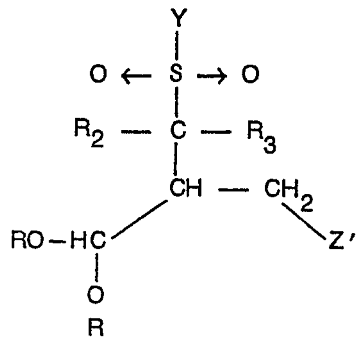

dans laquelle Y représente un reste aromatique, $R_2$ et $R_3$ représentent un radical alcoyle linéaire ou ramifié comportant de 1 à 4 atomes de carbone ou $R_2$ et $R_3$ représentent ensemble avec l'atome de carbone auquel ils sont liés un homocycle carboné comportant de 3 à 6 atomes de carbone, R représente un radical alcoyle comportant de 1 à 6 atomes de carbone ou bien les 2 radicaux R représentent ensemble une chaîne polyméthylène comportant 2 ou 3 atomes de carbone, et Z' représente un groupement $CO_2H$ ou un groupement Z, Z représentant un radical cyano ou un groupement $CO_2R_1$, $R_1$ représentant un radical alcoyle comportant de 1 à 6 atomes de carbone.

2. Les composés tels que définis à la revendication 1, répondant à la formule ($I_A$):

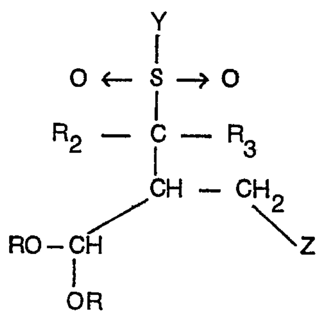

($I_A$)

dans laquelle Y, Z, R, $R_2$ et $R_3$ conservent les mêmes significations que dans la revendication 1.

3. Les composés tels que définis à la revendication 1, répondant à la formule ($I_B$):

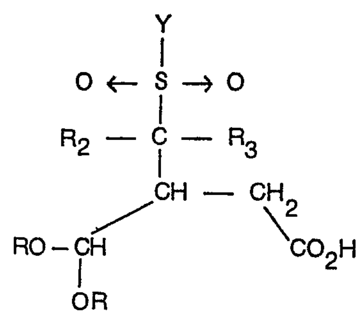

($I_B$)

dans laquelle Y, R, $R_2$ et $R_3$ conservent les mêmes significations que dans la revendication 1.

4. Les composés tels que définis à la revendication 1, 2 ou 3, caractérisés en ce que Y représente un radical phényle, tolyle ou xylyle.

5. Procédé de préparation des composés définis à l'une quelconque des revendications 1 à 4, caractérisé en ce que l'on fait réagir, en présence d'une base forte, au sein d'un solvant polaire, un composé de formule générale (II):

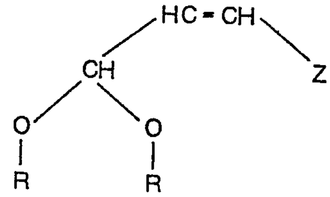

(II)

dans laquelle Z et R conservent les mêmes significations que dans la revendication 1, avec une sulfone de formule (III):

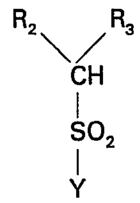

(III)

dans laquelle $R_2$, $R_3$ et Y conservent les mêmes significations que dans la revendication 1, pour obtenir un composé de formule générale ($I_A$):

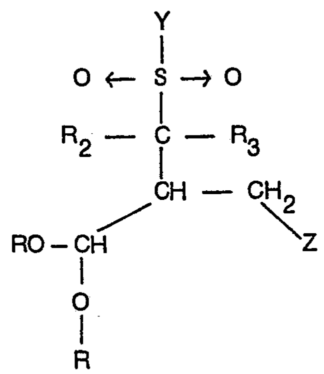

($I_A$)

dans laquelle Y, Z, R, $R_2$ et $R_3$ conservent les mêmes significations que dans la revendication 1, puis, si désiré, fait réagir sur le composé de formule ($I_A$)
—*soit*, lorsque Z représente un groupe —$CO_2R_1$, dans lequel $R_1$ est tel que défini à la revendication 1, un agent basique susceptible de saponifier le groupement —$CO_2R_1$ pour obtenir l'acide correspondant de formule ($I_B$):

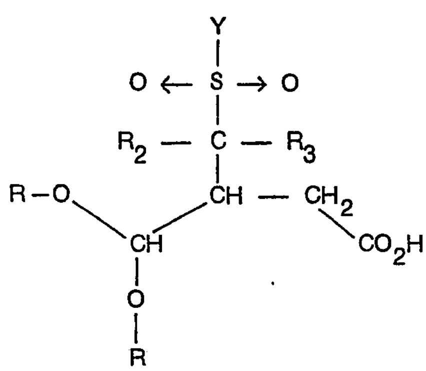

($I_B$)

dans laquelle Y, R, $R_2$ et $R_3$ conservent les significations précitées,
— *soit*, lorsque Z représente un groupement cyano, un agent d'hydrolyse susceptible de transformer le cyano en acide, pour obtenir également l'acide correspondant de formule ($I_B$).

6. Procédé de préparation selon la revendication 5, caractérisé en ce que la base forte en présence de laquelle on fait réagir le composé (II) avec le composé (III), est choisie dans le groupe constitué par les alcoolates alcalins, les hydrures alcalins, les amidures alcalins, le phényl lithium et les alcoyls lithiens.

7. Procédé de préparation selon la revendication 5, caractérisé en ce que le solvant polaire au sein duquel on fait réagir le composé de formule (II) et le composé de formule (III) est choisi dans le groupe constitué par le diméthylsulfoxyde, le diméthoxyéthane, le diméthylformamide, le tétrahydrofuranne, l'hexaméthylphosphorotriamide utilisé pur ou en mélange avec des hydrocarbures aromatiques monocycliques ou des cycloalcanes et un mélange de ces solvants.

8. Procédé selon l'une quelconque des revendications 5 à 7, caractérisé en ce que l'agent basique susceptible de saponifier le groupement —$CO_2R_1$ pour obtenir le composé de formule ($I_B$) est un

hydroxyde alcalin en solution hydroalcoolique.

9. Procédé selon l'une quelconque des revendications 5 à 7, caractérisé en ce que l'agent d'hydrolyse du groupement cyano du composé de formule ($I_A$) est un hydroxyde alcalin en solution hydroalcoolique.

10. Application des composés définis à l'une quelconque des revendications 1 à 4, qui consiste à transformer le composé de formule ($I_A$) en composé de formule ($I_B$) selon le procédé défini à la revendication 5 et est caractérisée en ce que l'on fait réagir sur le composé de formule ($I_B$) un agent acide susceptible d'hydrolyser la fonction cétal de la molécule, obtient ainsi un composé de formule (IV):

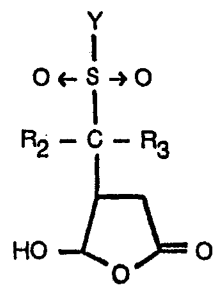

(IV)

et le soumet à l'action d'un agent basique pour obtenir l'acide 3-formyl 4-$R_2$ $R_3$ but-3-èn-1-oïque.

11. Application selon la revendication 10, caractérisée en ce que l'agent susceptible d'hydrolyser la fonction cétal de composé de formule ($I_B$) est un acide fort utilisé à l'état de traces, choisi dans le groupe constitué par l'acide sulfurique, l'acide chlorhydrique et l'acide paratoluène sulfonique, ledit acide fort étant utilisé en milieu acétonique et aqueux.

12. Application selon la revendication 10, caractérisé en ce que l'agent basique que l'on fait agir sur le composé de formule (IV) est un carbonate alcalin.

**Revendications pour l'Etat contractant: AT**

1. Procédé de préparation de l'acide 3-formyl but-3-èn-1-oïque

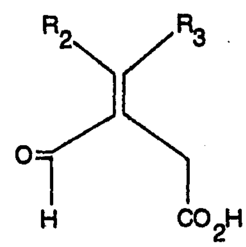

dans laquelle $R_2$ et $R_3$ représentent un radical alcoyle linéaire ou ramifié comportant de 1 à 4 atomes de carbone ou $R_2$ et $R_3$ représentent ensemble, avec l'atome de carbone auquel ils sont liés, un homocycle carboné comportant de 3 à 6 atomes de carbone, caractérisé en ce que l'on fait réagir, en présence d'une base forte, au sein d'un solvant polaire, un composé de formule générale (II):

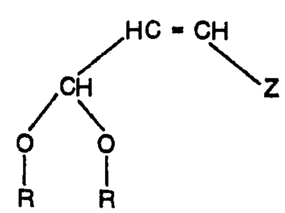

(II)

dans laquelle Z représente un groupement cyano ou un groupement $—CO_2R_1$, $R_1$ étant un radical alcoyle comportant de 1 à 6 atomes de carbone, R représente un radical alcoyle comportant de 1 à 6 atomes de carbone ou bien les deux radicaux R représentent ensemble une chaîne polyméthylène comportant 2 ou 3 atomes de carbone, avec une sulfone de formule (III):

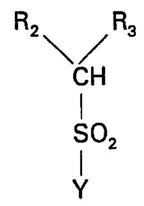

(III)

dans laquelle Y représente un reste aromatique, pour obtenir un composé de formule générale ($I_A$):

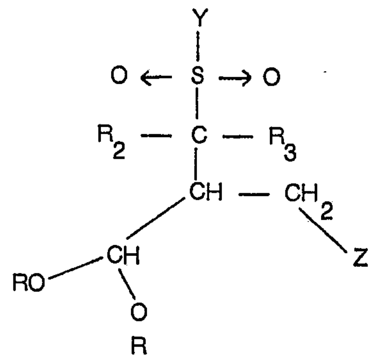

($I_A$)

dans laquelle Y, Z, R, $R_2$ et $R_3$ conservent les significations précitées, fait réagir sur le composé ($I_A$), *soit* lorsque Z représente un groupe $—CO_2R_1$, un agent basique susceptible de saponifier le groupement $—CO_2R_1$ pour obtenir l'acide correspondant de formule ($I_B$):

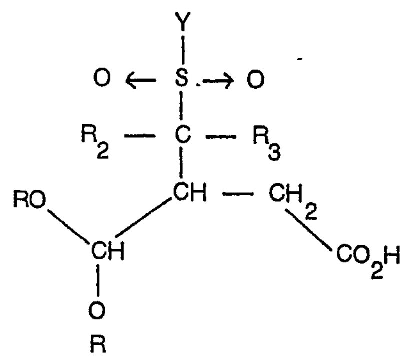

($I_B$)

dans laquelle Y, R, $R_2$ et $R_3$ conservent les significations précitées,
*soit* lorsque Z représente un groupement cyano, un agent d'hydrolyse susceptible de transformer, le cyano en acide, pour obtenir également l'acide de formule ($I_B$), fait réagir sur le composé de formule ($I_B$) un agent acide susceptible d'hydrolyser la fonction cétal de la molécule, obtient un composé de formule (IV):

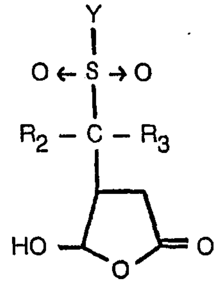

(IV)

et le soumet à l'action d'un agent basique pour obtenir l'acide 3-formyl but-3-èn-1-oïque désiré.

2. Procédé de préparation de l'acide 3-formyl but-3-èn-1-oïque, selon la revendication 1, caractérisé en ce que le substituant Y de la formule (III) représente un radical phényle, tolyle ou xylyle.

3. Procédé de préparation selon la revendication 1 ou 2, caractérisé en ce que la base forte en présence de laquelle on fait réagir le composé (II) avec le composé (III), est choisie dans le groupe constitué par les alcoolates alcalins, les hydrures alcalins, les amidures alcalins, le phényl lithium et les alcoyls lithiens.

4. Procédé de préparation selon la revendication 1, 2 ou 3, caractérisé en ce que le solvant polaire au sein duquel on fait réagir le composé de formule (II) et le composé de formule (III) est choisi dans le groupe constitué par le diméthylsulfoxyde, le diméthoxyéthane, le diméthylformamide, le tétrahydrofuranne, l'hexaméthylphosphorotriamide utilisé pur ou en mélange avec des hydrocarbures aromatiques monocycliques ou des cycloalcanes et un mélange de ces solvants.

5. Procédé selon l'une quelconque des revendications 1 à 4, caractérisé en ce que l'agent basique susceptible de saponifier le groupement —$CO_2R_1$ pour obtenir le composé de formule ($I_B$) est un hydroxyde alcalin en solution hydroalcoolique.

6. Procédé selon l'une quelconque des revendications 1 à 5, caractérisé en ce que l'agent d'hydrolyse du groupement cyano du composé de formule ($I_A$) est un hydroxyde alcalin en solution hydroalcoolique.

7. Procédé selon l'une quelconque des revendications 1 à 6, caractérisé en ce que l'agent susceptible d'hydrolyser la fonction cétal du composé ($I_B$) est un acide fort utilisé à l'état de traces, choisi dans le groupe constitué par l'acide sulfurique, l'acide chlorhydrique et l'acide paratoluènesulfonique, ledit acide fort étant utilisé en milieu acétonique et aqueux.

8. Procédé selon l'une quelconque des revendications 1 à 7, caractérisé en ce que l'agent basique que l'on fait agir sur le composé de formule (IV) est un carbonate alcalin.

**Claims for the Contracting States: BE CH DE GB IT LI LU NL SE**

1. The compounds with the formula:

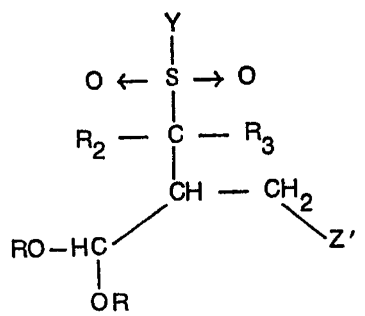

in which Y represents an aromatic residue, $R_2$ and $R_3$ represent each a linear or branched alkyl radical containing from 1 to 4 carbon atoms, or $R_2$ and $R_3$ together with the carbon atom to which they are bonded represent a carbon homocycle containing from 3 to 6 carbon atoms, R represents an alkyl radical containing from 1 to 6 carbon atoms or else the two R radicals together represent a polymethylene chain containing 2 or 3 carbon atoms and Z' represents a $CO_2H$ group or a group Z, Z representing a cyano radical or a group $CO_2R_1$, $R_1$ representing an alkyl radical containing from 1 to 6 carbon atoms.

2. The compounds as defined in Claim 1, corresponding to the formula ($I_A$):

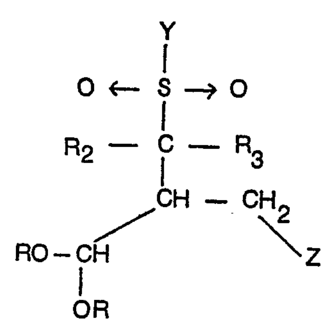

($I_A$)

in which Y, Z, R, $R_2$ and $R_3$ retain the same significances as in Claim 1.

3. The compounds as defined in Claim 1, corresponding to the formula ($I_B$):

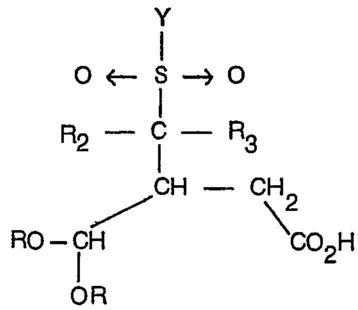

($I_B$)

in which Y, R, $R_2$ and $R_3$ retain the same significances as in Claim 1.

4. The compounds as defined in Claim 1, 2, or 3, characterized in that Y represents a phenyl, tolyl or xylyl radical.

5. Preparation process for the compounds defined in any one of the Claims 1 to 4, characterized in that a compound with the general formula (II):

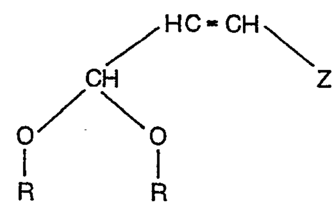

(II)

in which Z and R retain the same significances as in Claim 1, is made to react, in the presence of a strong base in a polar solvent, with a sulphone with the formula (III):

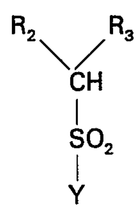

(III)

in which $R_2$, $R_3$ and Y retain the same significances as in Claim 1, so as to obtain a compound with the general formula ($I_A$):

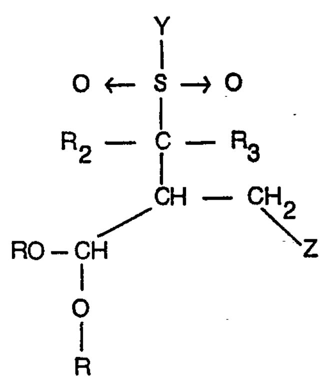

($I_A$)

in which Y, Z, R, $R_2$ and $R_3$ retain the same significances as in Claim 1, then, if desired, there is made to react on the compound with the formula ($I_A$),

— *either,* when Z represents a group —$CO_2R_1$, in which $R_1$ is as defined in Claim 1, a basic agent able

to saponify the $—CO_2R_1$ group, so as to obtain the acid corresponding to formula ($I_B$):

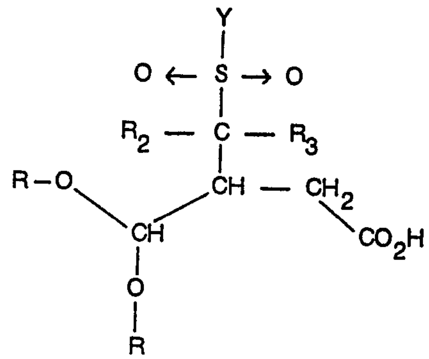

($I_B$)

in which Y, R, $R_2$ and $R_3$ retain the previously stated significances,
— *or*, when Z represents a cyano group, a hydrolyzing agent able to convert the cyano into acid, so also to obtain the acid corresponding to formula ($I_B$).

6. Preparation process according to Claim 5, characterized in that the strong base in the presence of which the compound (II) is made to react with the compound (III) is chosen from the group constituted by the alkaline alcoholates, the alkaline hydrides, the alkaline amides, phenyl-lithium and the alkyl-lithiums.

7. Preparation process according to Claim 5, characterized in that the polar solvent in which the compound with the formula (II) and the compound with the formula (III) are made to react, is chosen from the group constituted by dimethylsulphoxide, dimethoxyethane, dimethylformamide, tetrahydrofuran, hexamethylphosphorotriamide, utilized pure or mixed with monocyclic aromatic hydrocarbons or cycloalkanes and a mixture of these solvents.

8. Process according to any one of Claims 5 to 7, characterized in that the basic agent able to saponfy the group $—CO_2R_1$ so as to obtain the compound with the formula ($I_B$) is an alkaline hydroxide in a water-alcohol solution.

9. Process according to any one of Claims 5 to 7, characterized in that the hydrolyzing agent of the cyano group of the compound with the formula ($I_A$) is an alkaline hydroxide in a water-alcohol solution.

10. Application of the compounds defined in any one of the Claims 1 to 4, consisting in converting the compound with the formula ($I_A$) into the compound with the formula ($I_B$) according to the process defined in Claim 5, and characterized in that an acid agent able to hydrolyze the ketal function of the molecuse is made to react on the compound with the formula ($I_B$), so obtaining the compound with the formula (IV):

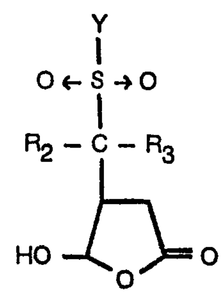

(IV)

and submitting it to the action of a basic agent so as to obtain 3-formyl 4-$R_2$ $R_3$ but-3-en-1-oic acid.

11. Application according to Claim 10, characterized in that the agent able to hydrolyze the ketal function of the compound with the formula ($I_B$) is a strong acid utilized in a trace state, chosen from the group constituted by sulphuric acid, hydrochloric acid and paratoluene sulphonic acid, the said strong acid being used in a water-acetone medium.

12. Application according to Claim 10, characterized in that the basic agent which is made to act on the compound with the formula (IV) is an alkaline carbonate.

**Claims for the Contracting State: AT**

1. Preparation process for 3-formyl but-3-en-1-oic acid

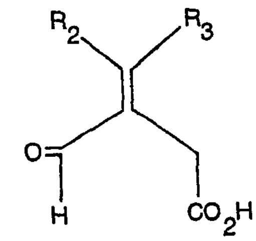

in which $R_2$ and $R_3$ represent a linear or branched alkyl radicals containing from 1 to 4 carbon atoms or $R_2$ and $R_3$, together with the carbon atom to which they are bonded, represent a carbon homocycle containing from 3 to 6 carbon atoms, characterized in that a compound with the general formula (II)

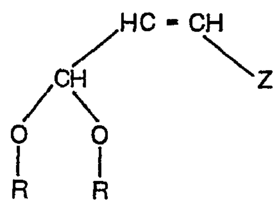

(II)

in which Z represents a cyano group or a group —$CO_2R_1$, $R_1$ being an alkyl radical containing from 1 to 6 carbon atoms, R represents an alkyl radical containing from 1 to 6 carbon atoms or else the two R radicals together represent a polymethylene chain containing 2 or 3 carbon atoms, is made to react, in the presence of a strong base in a polar solvent, with a sulphone with the formula (III):

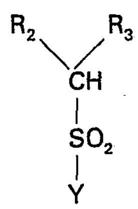

in which Y represents an aromatic residue, so as to obtain a compound with the general formula ($I_A$):

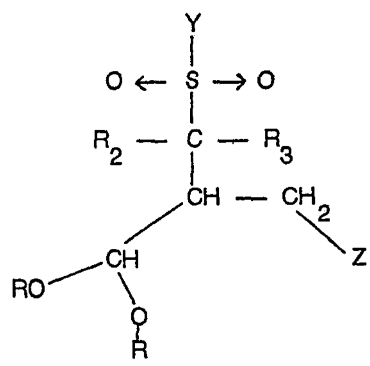

($I_A$)

in which Y, Z, R, $R_2$ and $R_3$ retain the significances previously stated, there is made to react on the compound ($I_A$),

— *either* when Z represents a group —$CO_2R_1$, a basic agent able to saponify the —$CO_2R_1$ group, so as to obtain the corresponding acid with the formula ($I_B$):

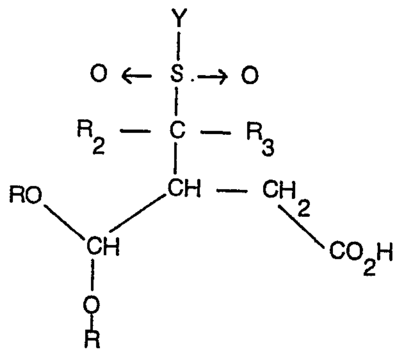

($I_B$)

in which Y, R, $R_2$ and $R_3$ retain the previously stated significances,

— *or* when Z represents a cyano group, a hydrolyzing agent able to convert the cyano into acid, so also to obtain the acid with the formula ($I_B$), an acid agent able to hydrolyze the ketal function of the molecule is made to react on the compound with the formula ($I_B$), a compound with the formula (IV):

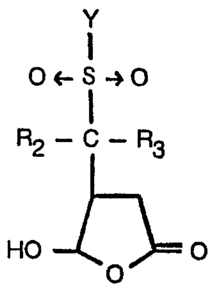

(IV)

is obtained and submitted to the action of a basic agent so as to obtain the desired 3-formyl but-3-en-1-oic acid.

2. Preparation process for 3-formyl but-3-en-1-oic acid according to Claim 1, characterized in that the substituent Y of the formula (III) represents a phenyl, tolyl or xylyl radical.

3. Preparation process according to Claim 1 or 2, characterized in that the strong base in the presence of which the compound (II) is made to react with the compound (III) is chosen from the group constituted by the alkaline alcoholates, the alkaline hydrides, the alkaline amides, phenyl-lithium and the alkyl-lithiums.

4. Preparation process according to Claim 1, 2 or 3, characterized in that the polar solvent in which the compound with the formula (II) and the compound with the formula (III) are made to react, is chosen from the group constituted by dimethylsulphoxide, dimethoxyethane, dimethylformamide, tetrahydrofuran, hexamethylphosphorotriamide utilized pure or mixed with monocyclic aromatic hydrocarbons or cycloalkanes and a mixture of these solvents.

5. Process according to any one of the Claims 1 to 4, characterized in that the basic agent able to saponify the group $—CO_2R_1$ so as to obtain the compound with the formula ($I_B$) is an alkaline hydroxide in a water-alcohol solution.

6. Process according to any one of the Claims 1 to 5, characterized in that the hydrolyzing agent of the cyano group of the compound with the formula ($I_A$) is an alkaline hydroxide in a water-alcohol solution.

7. Process according to any one of the Claims 1 to 6, characterized in that the agent able to hydrolyze the ketal function of the compound ($I_B$) is a strong acid utilized in a trace state, chosen from the group constituted by sulphuric acid, hydrochloric acid and paratoluene sulphonic acid, the said strong acid being used in a water-acetone medium.

8. Process according to any one of the Claims 1 to 7, characterized in that the basic agent which is made to act on the compound with the formula (IV) is an alkaline carbonate.

**Patentansprüche für die Vertragsstaaten: BE CH DE GB IT LI LU NL SE**

1. Verbindungen der Formel

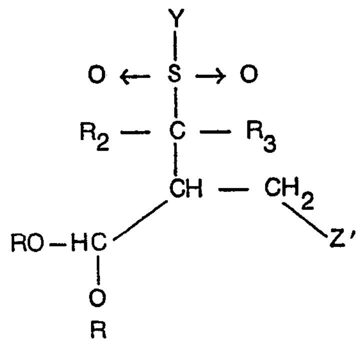

worin Y einen aromatischen Rest bedeutet, $R_2$ und $R_3$ einen linearen oder verzweigten Alkylrest mit 1 bis 4 Kohlenstoffatomen bedeuten, oder $R_2$ und $R_3$ gemeinsam mit dem Kohlenstoffatom, an das sie gebunden sind, einen Kohlenstoffhomocyclus mit 3 bis 6 Kohlenstoffatomen bilden, R einen Alkylrest mit 1 bis 6 Kohlenstoffatomen bedeutet oder die zwei Reste R gemeinsam eine Polymethylenkette mit 2 oder 3 Kohlenstoffatomen bilden und Z′ eine Gruppe $CO_2H$ oder eine Gruppe Z bedeutet, wobei Z einen Cyanorest oder eine Gruppe $CO_2R_1$, worin $R_1$ einen Alkylrest mit 1 bis 6 Kohlenstoffatomen darstellt, bedeutet.

2. Verbindungen gemäß Anspruch 1 der Formel $I_A$

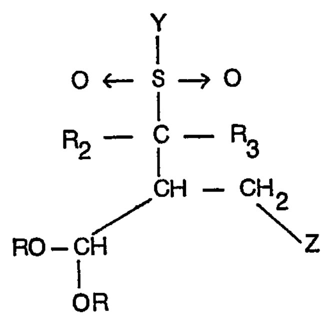

($I_A$)

worin Y, Z, R, $R_2$ und $R_3$ die in Anspruch 1 angegebenen Bedeutungen besitzen.

3. Verbindungen gemäß Anspruch 1 der Formel $I_B$

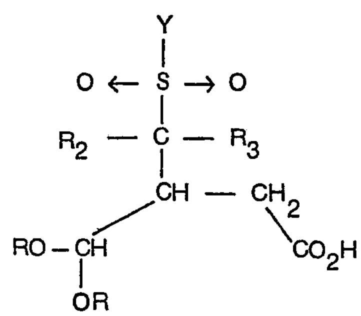

($I_B$)

worin Y, R, $R_2$ und $R_3$ die in Anspruch 1 angegebenen Bedeutungen besitzen.

4. Verbindungen gemäß Anspruch 1, 2 oder 3, dadurch gekennzeichnet, daß Y einen Phenyl-, Tolyl- oder Xylylrest bedeutet.

5. Verfahren zur Herstellung der Verbindungen gemäß einem der Ansprüche 1 bis 4, dadurch gekennzeichnet, daß man in Gegenwart einer starken Base in dem Medium eines polaren Lösungsmittels eine Verbindung der allgemeinen Formel II

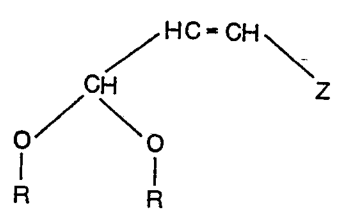

(II)

worin Z und R die in Anspruch 1 angegebenen Bedeutungen besitzen, mit einem Sulfon der Formel III

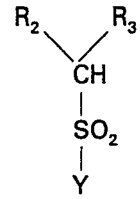

(III)

worin $R_2$, $R_3$ und Y die in Anspruch 1 angegebenen Bedeutungen besitzen, umsetzt, um eine Verbindung der allgemeinen Formel $I_A$

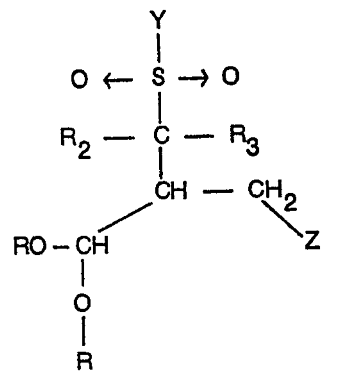

($I_A$)

zu erhalten, worin Y, Z, R, $R_2$ und $R_3$ die in Anspruch 1 angegebenen Bedeutungen besitzen und danach gewünschtenfalls mit der Verbindungen der Formel $I_A$
*entweder*, wenn Z eine Gruppe $—CO_2R_1$ bedeutet, worin $R_1$ wie in Anspruch 1 definiert ist, ein basisches Mittel, das befähigt ist, die Gruppe $—CO_2R_1$ zu verseifen, umsetzt, um die entsprechende Säure der Formel $I_B$

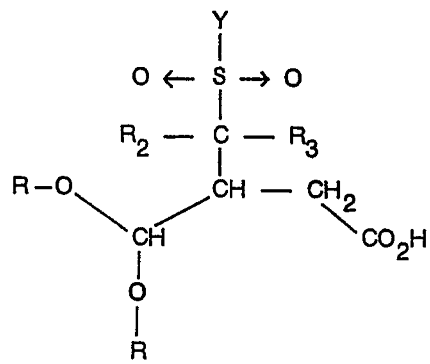

($I_B$)

zu erhalten, worin Y, R, $R_2$ und $R_3$ die vorstehend angegebenen Bedeutungen besitzen,
*oder*, wenn Z eine Cyanogruppe bedeutet, ein Hydrolysemittel umsetzt, das befähigt ist, die Cyanogruppe in die Säuregruppe zu überführen, um gleichfalls die entsprechende Säure der Formel $I_B$ zu erhalten.

6. Verfahren gemäß Anspruch 5, dadurch gekennzeichnet, daß die starke Base, in deren Gegenwart man die Verbindung II mit der Verbindung III umsetzt, ausgewählt wird unter den Alkalialkoholaten, den Alkalihydriden, den Alkaliamiden, Phenyllithium und den Alkyllithiumverbindungen.

7. Verfahren gemäß Anspruch 5, dadurch gekennzeichnet, daß das polare Lösungsmittel, in dessen Medium man die Verbindung der Formel II und die Verbindung der Formel III umsetzt, ausgewählt wird unter Dimethylsulfoxid, Dimethoxyäthan, Dimethylformamid, Tetrahydrofuran, Hexamethylphosphortrisamid, verwendet in reiner Form oder im Gemisch mit monocyclischen aromatischen Kohlenwasserstoffen oder Cycloalkanen, und einem Gemisch dieser Lösungsmittel.

8. Verfahren gemäß Anspruch 5 bis 7, dadurch gekennzeichnet, daß das basische Mittel, das befähigt ist, die Gruppe $—CO_2R_1$ zu verseifen, um die Verbindung der Formel $I_B$ zu ergeben, ein Alkalihydroxid in wäßrig alkoholischer Lösung ist.

9. Verfahren gemäß einem der Ansprüche 5 bis 7, dadurch gekennzeichnet, daß das Hydrolysemittel für die Cyanogruppe der Verbindung der Formel $I_A$ ein Alkalihydroxid in wäßrig alkoholischer Lösung ist.

10. Verwendung der Verbindungen gemäß einem der Ansprüche 1 bis 4, darin bestehend, daß man die Verbindung der Formel $I_A$ in die Verbindung der Formel $I_B$ gemäß dem in Anspruch 5 definierten Verfahren überführt und dadurch gekennzeichnet, daß man mit der Verbindung der Formel $I_B$ ein saures Mittel umsetzt, das befähigt ist, die Ketalfunktion des Moleküls zu hydrolysieren, so eine Verbindung der Formel IV

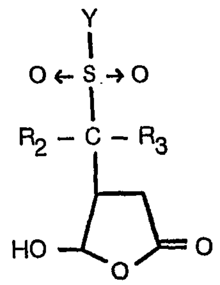

(IV)

erhält und diese der Einwirkung eines basischen Mittels unterzieht, um die 3-Formyl-4-$R_2$,$R_3$-but-3-en-1-säure zu erhalten.

11. Verwendung gemäß Anspruch 10, dadurch gekennzeichnet, daß das Mittel, das zur Hydrolyse der Ketalfunktion der Verbindung der Formel $I_B$ befähigt ist, eine starke Säure ist, die in Form von Spuren verwendet wird, ausgewählt unter Schwefelsäure, Chlorwasserstoffsäure und p-Toluolsulfonsäure, wobei die starke Säure in acetonischem und wäßrigem Milieu verwendet wird.

12. Verwendung gemäß Anspruch 10, dadurch gekennzeichnet, daß das basische Mittel, das man mit der Verbindung der Formel IV umsetzt, ein Alkalicarbonat ist.

**Patentansprüche für den Vertragsstaat: AT**

1. Verfahren zur Herstellung der 3-Formyl-but-3-en-1-säure

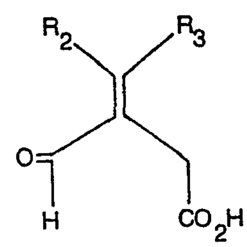

worin $R_2$ und $R_3$ einen linearen oder verweigten Alkylrest mit 1 bis 4 Kohlenstoffatomen darstellen oder $R_2$ und $R_3$ gemeinsam mit dem Kohlenstoffatom, an das sie gebunden sind, einen Kohlenstoffhomocyclus mit 3 bis 6 Kohlenstoffatomen bilden, dadurch gekennzeichnet, daß man in Gegenwart einer starken Base in dem Medium eines polaren Lösungsmittels eine Verbindung der allgemeinen Formel II

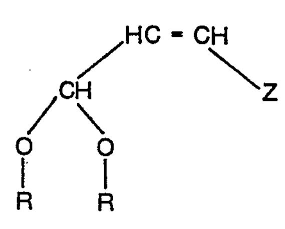

(II)

worin Z eine Cyanogruppe oder eine Gruppe —$CO_2R_1$, worin $R_1$ einen Alkylrest mit 1 bis 6 Kohlenstoffatomen darstellt, bedeutet, R einen Alkylrest mit 1 bis 6 Kohlenstoffatomen bedeutet oder die beiden Reste R gemeinsam eine Polymethylenkette mit 2 oder 3 Kohlenstoffatomen bilden, mit einem sulfon der Formel III

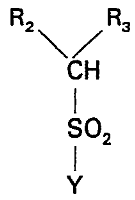

(III)

worin Y einen aromatischen Rest bedeutet, umsetzt, um eine Verbindung der allgemeinen Formel $I_A$

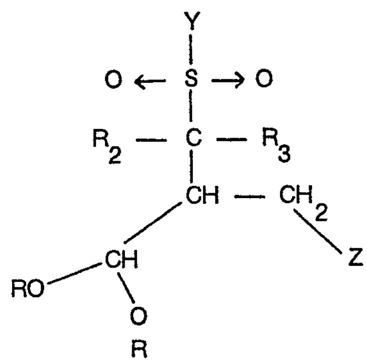

($I_A$)

zu erhalten, worin Y, Z, R, $R_2$ und $R_3$ die vorstehend angegebenen Bedeutungen besitzen, mit der Verbindung IA
*entweder,* wenn Z eine Gruppe $-CO_2R_1$ bedeutet, ein basisches Mittel, das befähigt ist, die Gruppe $-CO_2R_1$ zu verseifen, umsetzt, um die entsprechende Säure der Formel $I_B$

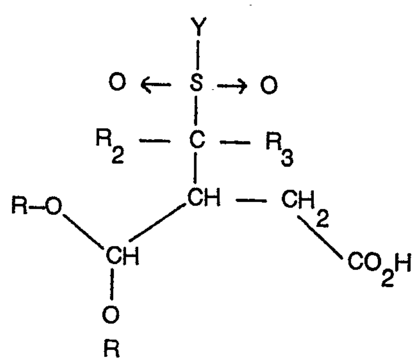

($I_B$)

zu erhalten, worin Y, R, $R_2$ und $R_3$ die vorstehend angegebenen Bedeutungen besitzen,
*oder,* wenn Z eine Cyanogruppe bedeutet, ein Hydrolysemittel, das befähigt ist, die Cyanogruppe in die Säuregruppe zu überführen, umsetzt, um gleichfalls die Säure der Formel $I_B$ zu erhalten, mit der Verbindung der Formel $I_B$ ein saures Mittel, das befähigt ist, die Ketalfunktion des Moleküls zu hydrolysieren, umsetzt, eine Verbindung der Formel IV

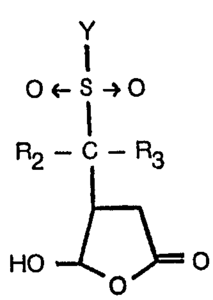

(IV)

erhält und diese der Einwirkung eines basischen Mittels unterzieht, um die gewünschte 3-Formyl-but-3-en-1-säure zu erhalten.

2. Verfahren zur Herstellung der 3-Formyl-but-3-en-1-säure gemäß Anspruch 1, dadurch gekennzeichnet, daß der Substituent Y der Formel III einen Phenyl-, Tolyl- oder Xylylrest darstellt.

3. Verfahren gemäß Anspruch 1 oder 2, dadurch gekennzeichnet, daß die starke Base, in deren Gegenwart man die Verbindung II mit der Verbindung III umsetzt, ausgewählt wird unter den Alkalialkoholaten, den Alkalihydriden, den Alkaliamiden, dem Phenyllithium und den Alkyllithiumverbindungen.

4. Verfahren gemäß Anspruch 1, 2 oder 3, dadurch gekennzeichnet, daß das polare Lösungsmittel, in dessen Medium man die Verbindung der Formel II und die Verbindung der Formel III umsetzt, ausgewählt wird unter Dimethylsulfoxid, Dimethoxyäthan, Dimethylformamid, Tetrahydrofuran, Hexamethylphosphortrisamid, verwendet in reiner Form oder im Gemisch mit monocyclischen aromatischen Kohlenwasserstoffen oder Cycloalkanen, und einem Gemisch dieser Lösungsmittel.

5. Verfahren gemäß einem der Ansprüche 1 bis 4, dadurch gekennzeichnet, daß das basische Mittel, das befähigt ist, die Gruppe $-CO_2R_1$ zu verseifen, um die Verbindung der Formel $I_B$ zu ergeben, ein Alkalihydroxid in wäßrig alkoholischer Lösung ist.

6. Verfahren gemäß einem der Ansprüche 1 bis 5, dadurch gekennzeichnet, daß das Hydrolysemittel für die Cyanogruppe der Verbindung der Formel $I_A$ ein Alkalihydroxid in wäßrig alkoholischer Lösung ist.

7. Verfahren gemäß einem der Ansprüche 1 bis 6, dadurch gekennzeichnet, daß das Mittel, das zur Hydrolyse der Ketalfunktion der Verbindung $I_B$ befähigt ist, eine starke Säure ist, die in Form von Spuren verwendet wird, ausgewählt unter der Schwefelsäure, der Chlorwasserstoffsäure und der p-Toluolsulfonsäure, wobei diese starke Säure in acetonischem und wäßrigem Milieu verwendet wird.

8. Verfahren gemäß einem der Ansprüche 1 bis 7, dadurch gekennzeichnet, daß das basische Mittel, das man mit der Verbindung der Formel IV umsetzt, ein Alkalicarbonat ist.